# EUROPEAN PATENT APPLICATION

(11) **EP 1 447 046 A1**
(43) Date of publication of application: **18.08.2004**
(21) Application number: 03007524.6
(22) Date of filing: 01.04.2003
(51) Int. Cl.: A61B 6/00, G01N 23/04, G01N 23/20

(54) **Apparatus and method to obtain phase contrast x-ray images**

(30) Priority: 14.02.2003 EP 30003389
(71) Applicant: PAUL SCHERRER INSTITUT, 5232 Villigen PSI (CH)
(72) Inventor: Christian, David, 79787 Lauchingen (DE)
(74) Representative: Fischer, Michael, Dr.

(57) **Abstract**

An apparatus (2) for generating a phase contrast x-ray image (4) comprising in an optical path (6) as seen in the direction of the light flow:
a) an incoherent x-ray source (8);
b) a first beam splitter grating (12) for splitting the light beams (14) of said x-ray source (8);
c) a second beam recombiner grating (20) for recombining the splitted beams (16, 18) in a recombination distance from the second beam recombiner grating (20);
d) an optional third analyzer grating (22) in order to offer an adsorption lines grating matching the interference lines downstream of said second beam recombiner grating (20) in an analyzer plane (a);
e) an image detector (30) disposed downstream of the analyzer plane (a); and
f) a means for introducing a sample (24) into said optical path (6) upstream or downstream of the second beam recombiner grating (20).

This apparatus is significantly improved insofar as virtually no longitudinal coherence is required and simultaneously spatially incoherent x-rays (i.e. radiation stemming from a relatively large source placed at a relatively short distance) can be used. This properties means that this set-up now is perfectly suited for use with a standard x-ray tube, opening this technique to a wide spread of medical and/or commercial applications.

## Description

The invention relates to an apparatus and a method to obtain phase contrast x-ray images and/or tomograms in medical, biological and/or material science applications.

In the hard x-ray region (photon energies > 8 keV) the phase-shift cross sections of samples having comparably low densities are much larger than the absorption cross-sections. As an example, a 50 µm thick organic object (polystyrene) only absorbs 0.2% at 20 keV, meaning that it is barely visible in absorption contrast as it has almost no effect on the amplitude of the transmitted light waves. This means, that a high dose of radiation is necessary to obtain an image with a suitable signal noise ratio. However, the same object shifts the phase of the transmitted wave by as much as 180°. If a wave with such a phase shift would interfere with an unshifted reference wave, it could cause an amplitude variation with a visibility of 1, meaning the resulting intensity in the interference region would be zero. The contrast caused by the object would thus be tremendeously higher than the absorption contrast, which in turn can be exploited to use much less radiation to give a good image.

The reduction of the applied radiation dose is of paramount interest in several medical applications, especially in mammography. Here, the difference in absorption between the abnormity (e.g. a breast cancer tumor) and the surrounding healthy tissue is very small compared to - for example - the case when a broken bone is investigated. As this difference in absorption becomes even less with increasing photon energy, comparatively low photon energies of 15 - 25 keV are applied in mammography. Although this energy is optimized to give the best possible trade-off between image quality and radiation damage, the absorbed dose is severe. This is especially problematic since mammography is often applied in screening tests, so that a large number of healthy patients is submitted to the high radiation dose. Even though the risk of radiation-induced cancer is lower than the benefit of such screening tests, it is known that a non-neglectable risk for initially healthy women exists.

Other medical applications that are problematic concerning their radiation load are such that require many images for an investigation. Among these are tomographic applications that typically require about 100 images to allow for a good 3-dimensional reconstruction, and monitoring applications that take film sequences using x-rays.

In addition to medical applications, any investigation of low contrast objects e.g. in biology or material science could benefit from exploiting the phase contrast. It should be mentioned, that an object imbedded in a matrix of the same absorption coefficient is completely invisible in absorption contrast, while for the same sample significant differences in phase shift may occur.

At optical or x-ray wavelengths, the phase of a wave cannot be measured directly, so any conversion of a phase shift into an intensity modulation has to depend on interference of two (or more) waves. In order to be able to interfere constructively or destructively, the waves need to have a well-defined phase relation in time and space, i.e. sufficient temporal (longitudinal) coherence and spatial (transverse) coherence.

Temporal coherence is related to the monochromaticity of the radiation. For radiation of a bandwidth of δλ around a central wavelength λ, the longitudinal coherence length is about λ²/δλ. Let us consider two beams stemming from the same source point that are superimposed after taking different paths through an optical set-up. These beams only have a well-defined phase relation if the difference in optical path lengths is shorter than the longitudinal coherence length. While for visible laser light λ²/δλ can extend over many meters, it is only in the order of a micron at hard x-ray wavelengths even when a crystal monochromator (λ/δλ ≈ 10⁴) is used.

Spatial coherence is related to the size and the distance of the radiation source, considering a source of a size *s* composed of independent (i.e. incoherent) monochromatic point sources emitting at a wavelength λ. If a look is taken at two points separated by a distance *d*, which lie inside a plane that is placed at a distance *l* from the source, the relative phase of the radiation light impinging on these two points will only be well defined, when the condition *d*<λ. *l*/*s* is fulfilled. *d* is called the transverse coherence length. If, for instance, two slits placed at a distance *l* from the source are used for an interference experiment, a clear diffraction pattern can only be obtained when the slit separation is smaller than *d*. For an x-ray tube placed at 1 m distance having a source spot of 0.1 mm emitting at λ=0.1 nm, the transverse coherence length is again in the order of only one micron.

For a given source both values, longitudinal and transverse coherence, can be increased only at cost of reduced photon flux density. Any optical device reducing the bandwidth will select a fraction of the available photons, and will thus reduce the flux density at least proportional to the gain in longitudinal coherence. The transverse coherence can be increased by increasing the source distance, which will lead to a loss in flux density proportional to the square to the gain in coherence. A reduction of the source size would improve the transverse coherence, but the limitations of power density in the anode material again will lead to a loss in flux density.

Intense hard x-rays with high temporal and spatial coherence can at present only be obtained from synchrotron sources. The use of such facilities is very costly and therefore only of little use for the purposes described above. The commercial impact of any invention in context with hard x-rays therefore strongly depends on whether it can be applied with x-rays from an x-ray tube. As the radiation from x-ray tubes is much less coherent compared to synchrotron radiation, the required degree of coherence is of crucial importance.

A variety of x-ray techniques have been developed to exploit the phase of a sample, i.e. to convert it into an amplitude contrast in the detector plane. An excellent review on these methods can be found under [1]. Fresnel diffraction of coherent hard x-rays at the edges of a phase object has been used to significantly improve the visibility of an object in microradiography. In first approximation, the obtained intensity distribution is proportional to the Laplacian (second derivative) of the refractive index distribution, and in certain cases a reconstruction of a phase object from a single micrograph is possible. Quantitative information on arbitrary phase objects can be obtained by numerically evaluating series of images acquired with the detector placed at different distances from the sample. Both high longitudinal and transverse coherence is required in this method, which is the reason for carrying out these experiments mainly at synchrotron radiation sources.

Only in a few cases, similar experiments have been carried out successfully using an x-ray tube with a microfocus source. Although these set-ups significantly improved the visibility of low absorbing specimens, they either required high transverse coherence [2], or high longtudinal coherence [3], leading to poor photon efficiency.

The most sensitive method to measure the phase shifts introduced to a wave front is interferometry. In analogy to visible light interferometers, one can distinguish two different types of set-ups. In the so called Mach-Zehnder type the incoming light is split into two separated branches of which one passes through the sample while the other serves as an unperturbed reference beam. The two beams interfering at the exit of the interferometer give an intensity distribution that represents the difference in optical path and thus - if perfectly aligned - of the phase shift caused by the object. In the common path type interferometer, the incoming beam is split into two beams with a small separation, which both pass along almost the same optical path.

A set-up for a Mach-Zehnder type interferometer operated in the hard x-ray range was introduced by Bonse and Hart more than three decades ago. It consists of three partially transmitting Bragg crystals used as beam splitter and beam recombining elements. Ando and Hosoya pioneered phase contrast imaging with such a device in the early seventies, and more recent set-ups have produced large numbers of excellent phase contrast images and computer tomograms, e.g. of biological specimens. The main technical difficulty lies in the extreme demands on the mechanical stability of the optical components, as the relative positions of the optical components have to be stable within a fraction of a lattice constant, i.e. to sub-Ångstrom dimensions. Therefore, Bonse-Hart interferometers are extremely difficult to handle especially when made big enough to investigate samples with dimensions of more than a few millimeters. The set-up was initially used with x-ray tube sources, and it requires little transverse coherence. However, the requirements with respect to the monochromaticity, i.e. longitudinal coherence are given by the applied Bragg crystals, which only accept a extremely narrow bandwidth. Therefore, only a minute fraction of the radiation emitted from the source contributes to the image.

The Bonse-Hart interferometer also has another severe drawback: as all Mach-Zehnder type interferometers, the intensity of the interference pattern depends on the relative phase shift of object in a non-linear and non-monotonic fashion: it oscillates between a minimum and a maximum value with a period of 2π in phase shift. Therefore thick samples, which cause a phase shift of multiples of 2π - as it is the case in most medical applications - will result in a confusing interferogram of fringes, which is very difficult to convert in a phase image, as there is no unique solution. So the fact that the set-up is so enormously sensitive to phase shifts at the same time limits its dynamic range considerably.

Recently, a common path interferometer for hard x-rays has been developed, in which the interfering beams are not completely separated but merely sheared by a small angle, so that they pass through different, closely spaced parts of the sample. Transmission diffraction gratings are used as optical elements made from silicon single crystals by electron beam lithography and anisotropic wet chemical etching. In the context the experimental results were obtained using an interferometer consisting of two phase gratings and a Bragg analyzer. This set-up requires both transverse coherence (due to the transverse separation of the interfering beam pairs) and was carried out using highly monochromatic radiation originating from synchroton sources because of the used Bragg analyzer.

For the reason given above, it is the aim of the invention to provide an apparatus and a method for obtaining phase contrast x-ray images in a more simplified manner which requires x-radiation of low temporal and spatial coherence.

With respect to the apparatus this aim is solved according to the invention by an apparatus for generating a phase contrast x-ray image comprising in an optical path as seen in the direction of the light flow:
a) an x-ray source providing radiation with low coherence;
b) a first beam splitter grating for splitting the light beams of said x-ray source;
c) a second beam recombiner grating for recombining the splitted beams in a recombination distance from the second grating;
d) a third analyzer grating in order to offer an adsorption line grating matching the interference lines downstream of said second beam recombiner grating in an analyzer plane;
e) an image detector placed downstream of the analyzer plane; and
f) a means for introducing a sample into said optical path upstream or downstream of the second beam recombiner grating.

It should be understood that instead of a separate third analyzer grating as mentioned under d) and a separate image detector as mentioned under e) even an image detector having a strip-type sensitivity for x-ray radiation is disclosed in interpretation of the claim language. This type of image detector may have a strip-type structure of phosphor or a strip-type pixel detector adapted to the orientation of the interference lines and their periodicity.

This apparatus is significantly improved insofar as virtually no longitudinal coherence is required and simultaneously x-rays with little spatial coherence (i.e. radiation stemming from a relatively large source placed at a relatively short distance) can be used. This properties means that this set-up now is perfectly suited for use with a standard x-ray tubes, opening this technique to a wide range of medical and commercial applications.

With respect to the method this aim is solved according to the invention by a method for generating a phase contrast x-ray image of a sample, comprising:
a) applying beams of an incoherent x-ray source to a first beam splitter grating in order to split the beams;
b) applying the splitted beams to a second beam recombiner grating in order to recombine the splitted beams in an analyzer plane;
c) introducing a sample either into the splitted beams between the first beam splitter grating and second beam recombiner grating or into the splitted beams between the second beam recombiner grating and the third analyzer grating; and
d) measuring the light intensity distribution in or after the analyzer plane.

The proposed set-up and method is described below explaining several additional but optional features.

A standard x-ray tube (stationary or rotating anode type) can be used as radiation source. The anode material should be chosen to have appropriate emission lines in the relevant energy range. For mammography applications these could be Nb, Mo, Rh, Pd, Ag, which have K-emission lines in the 5 to 50 keV, preferably 15 to 25 keV region. Especially suited for this set-up would be a line focus tube. In this case, the grating lines should be parallel to the source line (if a line focus source is used), as the setup requires even less coherence in the direction along the lines. The spectrum of the x-ray source may be filtered to reduce the source bandwidth.

At a convenient distance *d*_{*1*} downstream of the source depending on the required field of view (0.1 m - 5 m, typically in the range of 0.5 to 2 m) a diffraction grating with a period *p*_{*1*} consisting of phase shifting material is placed. This first beam splitter grating splits the incoming radiation into essentially two diffraction orders, preferentially the +1st and -1st order. The diffraction angle between these two orders is given by approximately *2*·λ/*p*, where λ is the wavelength and *p* is the grating period.

In a distance *d*_{*2*} of typically 0.01 to 1 m, preferably 0.05 to 0.5 m, a second beam recombiner phase grating with half the period of the first grating *p*_{*2*} = *p*_{*1*}/*2* and parallel alignment is placed. At this point, each beam that passed through the first beam splitter grating is separated by *s* = *2·λlp*_{*1*}*· d*_{*2*}. As this the second beam recombiner grating causes twice the diffraction angle, it recombines the two diffraction orders in a distance d₃ in the analyser plane downstream of the second beam recombiner grating, whereas the distance of this analyser plane has to be chosen substantially as *d*_{*3*} *= d*_{*2*}*.*

The two beams interfere in the analyser plane and cause a field of standing waves, i.e. a field of interference lines with high and low intensity parallel to the grating lines and a periodicity equal to that of the second beam recombiner grating. The most important aspect of this invention is the fact that the interference lines occur even when the radiation is spatially incoherent (i.e. coming from large source at close distance) and temporally incoherent (i.e. polychromatic). The lines only occur in certain planes, namely when the conditions *d*_{*2*} *= d*_{*3*} is at least approximately fulfilled. Furthermore, the condition *p*_{*2*} *= p*_{*1*}/*2* has to be at least approximately fulfilled.

In this analyser plane a third analyser grating may be placed that can consist of highly absorbing lines with the same periodicity as the interference lines *(p*_{*3*} *= p*_{*2*}*).* The orientation of the analyzer grating lines should be parallel to the interference lines. If a sample is placed somewhere between the first beam splitter grating and the third analyzer grating both interfering beams will be shifted in phase by the sample if a difference in phase shift, that means a phase gradient of the sample, occurs. This difference in phase shift causes the positions of the interference lines in the analyzer plane to be shifted. By this shift an intensity change of the transmitted light will occur. The recorded images which are taken with a x-ray image detector are therefore differential phase images. This differential phase images can be used directly to enhance the visibility of phase shifting objects, or they can be converted into phase images by integration of the image intensity values perpendicular to the grating line orientation.

The separation of the two beams in the sample plane determines the sensitivity of the apparatus set-up, i.e. the intensity change caused by a given phase gradient in the sample.

The position of the sample along the optical axis and the grating distances can be chose within certain limits. This can be advantageous to select the optimum sensitivity, as phase shift differences between two interfering beams of more than π may cause difficulties in the reconstruction of the phase image by integration. Typical values for the grating periods would be in the range of 0.1 to 100 micron, preferably 1 to 10 micron, for a wavelength of λ = 0.05 nm (24.8 keV) and grating distances of 10 cm, the corresponding separation would be in the range of 1 to 10 microns.

The height of the phase shifting grating structures should be optimized to give maximum diffraction efficiency of the +1st and -1st diffraction order and to minimize other diffraction orders. Good values for the phase shift of the structures are close to π (or odd multiples of π). The grating duty cycle should be close to 0.5 and the absorption should be low to minimize the zero order radiation.

In the relevant x-ray range the necessary structure heights are typically in the range of tens of microns. It is therefore necessary to choose the grating material with regard to a suitable fabrication process. A material well suited for the fabrication of such phase gratings is silicon. For this material, the height for π phase shift is 19 µm, 25.5 µm, and 32 µm for 15 keV, 20 keV and 25 keV photon energy, respectively. Silicon can be patterned fairly easily with high aspect ratios using photo- or electron beam lithography and wet etching or dry etching techniques, which are standard methods in microfabrication. Other materials well suited for this purpose are polymers, or metals such as Copper or Nickel, which can be patterned by electroplating.

The absorbing analyzer gratings can be made of heavy elements such as Tungsten, Tantal, Molybdenum (by dry etching) or Gold (by electroplating).

The afore mentioned invention therefore unites the following properties and advantages:
a) The setup comprising the beam splitter grating and the beam recombiner grating is achromatic and requires practically no spatial coherent light. The setup can be operated therefore with a standard x-ray tube.
b) The setup is less sensitive to phase shifts than Mach-Zehnder type interferometers. Therefore, the setup allows to investigate samples that cause large phase shifts.
c) The phase shift sensitivity can be tuned within certain limits by changing the sample position or the grating positions or by using grating sets with different periods.
d) The related materials and fabrication methods for the gratings are comparably well-known and can be easily performed.
e) The setup is very well suited for mammography for the reasons given in the introduction (suitable energy range, screening applications, radiation damage).
f) The setup is very well suited for applications that require many x-ray images (tomography, series of x-ray images etc.).

Other advantageous optional features of the invention can be found in the other depending claims.

The invention is exemplarily described according to the drawings below. They show in:
- Figure 1: a schematic view on an apparatus for obtaining phase contrast x-ray images; and
- Figure 2: an interferogram of 100 and 200 µm diameter polystyrene spheres; a) showing the differential phase image and b) showing the integrated phase image.

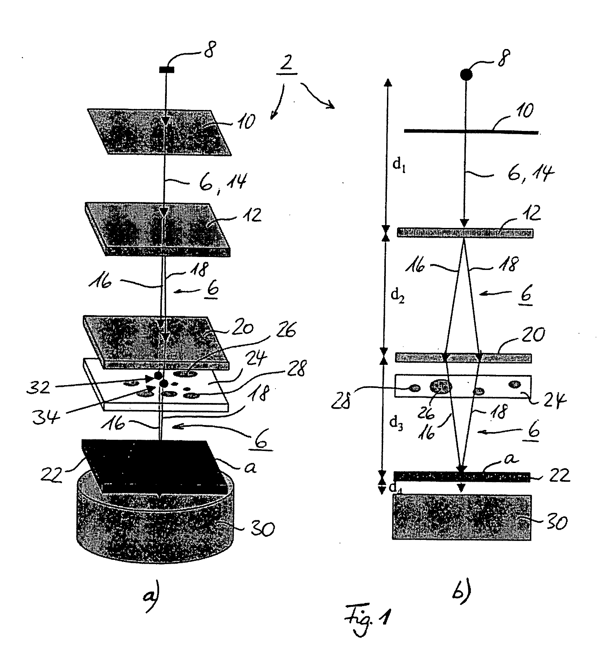

Figur 1 shows a schematic view on an apparatus 2 for obtaining phase contrast x-ray images 4. Part a) of figure 1 shows a schematic 3-dimensional view on the apparatus 2 and part b) a lateral view respectively.

In an optical path 6 a standard x-ray tube 8 is used as radiation source. The anode material of the x-ray tube 8 has emission lines in the energy range of 20 keV which is suitable for mammography applications. Typical anode materials are Nb, Mo, Rh, Pd, Ag, which have K-emission lines in the 15 to 25 keV region. Especially suited for this apparatus 2 is a line focus tube. In this case, the grating lines of the latter gratings are parallel to the source line, as the apparatus requires even less coherence in the direction along the lines. The spectrum of the x-ray tube 8 is filtered by a filter 10 to reduce the source bandwidth which is not necessarily required.

At a distance *d*_{*1*} = 1 m downstream of the x-ray tube 8 a first diffraction grating with a period *p*_{*1*} consisting of phase shifting material is placed. This first beam splitter grating 12 splits the incoming filtered radiation beam 14 into essentially two diffraction orders 16 and 18, which are in fact the +1st and -1st order. The diffraction angle between these two orders are given by approximately *2·λ*/*p,* where λ is the wavelength and *p* is the grating period.

In a distance *d*_{*2*} = 0.5 m, a second beam recombiner grating 20 with half the period of the first beam splitter grating 12 having *p*_{*2*} = *p*_{*1*}/*2* and parallel alignment is placed. At this point, each beam 14 that passed through the first beam splitter grating 12 is separated by *s* = *2·λ*/*p*_{*1*}*·d*_{*2*}*.* As this the second beam recombiner grating 20 causes twice the diffraction angle, it recombines the two diffraction order beams 16, 18 in a distance d₃ = 0.5 m downstream of the second beam recombiner grating 20 in an analyser plane a.

The two beams 16, 18 interfere in the analyser plane a that is in a distance d₃ downstream of the second beam recombiner grating 20 and cause a field ofinterference lines. The most important aspect of this invention is the fact that the interference lines occur even when spatiallly incoherent and temporally incoherent (i.e. polychromatic) x-ray tube 8 is used.

In this plane a third analyser grating 22 is placed that consist of highly absorbing lines with the same periodicity as the interference lines *(p*_{*3*} *= p*_{*2*}*).* The orientation of the analyzer grating lines is parallel to the interference lines.

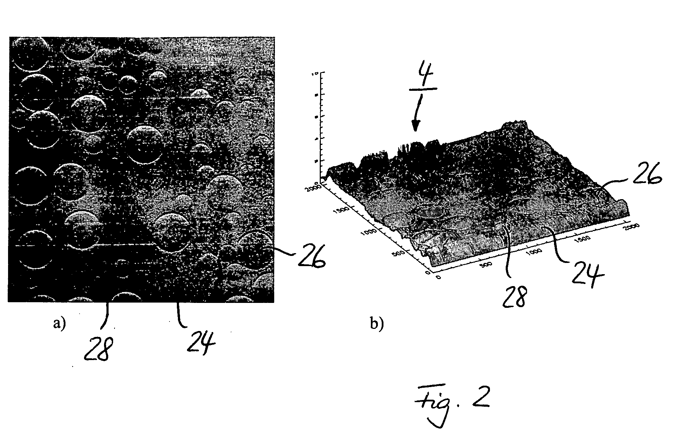

A sample 24 having different spheres 26, 28 is placed between the first beam splitter grating 12 and the third analyzer grating 22, i.e. in a region where the beams 16, 18 are split, both interfering beams 16, 18 will be shifted in phase by the sample 24. If there is a difference in phase shift (i.e. a phase gradient of the sample 24, in this example caused by the spheres 26, 28) the position of the interference lines in the analyzer plane a is shifted. By this shift an intensity change of the transmitted light will occur. The recorded images are therefore differential phase images as shown in part a) of figure 2. This differential phase images can either be used directly to enhance the visibility of a phase shifting object, or it may be converted into a phase image 4 by integration of the image intensity values perpendicular to the grating line orientation.

The separation of the two beams 16, 18 in the plane of the sample 24 indicated by dots 32, 34 determines the sensitivity of the apparatus set-up, i.e. the intensity change caused by a given phase gradient in the sample 24. The position of the sample 24 along the optical path 6 and the grating distances can be chose within certain limits. This can be advantageous to select the optimum sensitivity, as phase shift differences between the two interfering beams 16, 18 of more than π may cause difficulties in the reconstruction of the phase image 4 by integration. Typical values for the grating periods are in the range of 1 to 10 micron, for a wavelength of λ = 0.05 nm (24.8 keV) and grating distances of 10 cm, the corresponding separation of the two beams 16, 18 would be in the range of 1 to 10 microns.

The height of the phase shifting grating structures should be optimized to give maximum diffraction efficiency of the +1st and -1st diffraction order and to minimize other diffraction orders. Good values for the phase shift of the structures are close to π (or odd multiples of π). The grating duty cycle should be close to 0.5 and the absorption should be low to minimize the zero order radiation.

In the relevant x-ray range the necessary structure heights are typically in the range of tens of microns. It is therefore necessary to choose the grating material with regard to a suitable fabrication process. A material well suited for the fabrication of such phase gratings is silicon. For this material, the height for π phase shift is 19 µm, 25.5 µm, and 32 µm for 15 keV, 20 keV and 25 keV photon energy, respectively. Silicon can be patterned fairly easily with high aspect ratios using photo- or electron beam lithography and wet etching or dry etching techniques, which are standard methods in microfabrication. The third analyzer grating 22 is made of gold in a dry etching step.

Figure 2 now depicts an interferogram of a sample having 100 and 200 µm diameter polystyrene spheres 28 resp. 26. This interferogram can be achieved with the apparatus 2 shown in figure 1. For the first beam splitter grating 12 and the second beam recombiner grating 20 a Si phase grating having a two micron period has been used. For the third analyser grating 22 a 1 micron period Au analyzer grating has been used. As x-ray radiation source a x-ray tube with 24.8 keV photon energy has been used. It can be seen in part a) of figure 2 that the spheres 26, 28 appear darker than the surrounding grey area on the lower edges and brighter on the upper edges. Part b) of figure 2 shows a numerical integration of the same differential image revealing the phase shift corresponding to the spherical shape of the phase shifting objects, here the spheres 26, 28. It should be noticed that the spheres 26, 28 would be practically invisible without the interferometer setup due to the very low absorption in the hard x-ray range.

### References

(1) R. Fitzgerald,
   *Phase-Sensi tive X-Ray Imaging*
   Physics Today **53**, No. 7 (2000) p. 23
   *http;*//*www.aip.org*/*pt*/*vol-53*/*iss-7*/*p23. html*
(2) S.W. Wilkins, T.E. Gurejev, D. Gao, T. Pogani, A.W. Stevenson
   *Phase-contrast imaging using polychromatic hard x-rays*
   Nature **384** (1996) p. 335-338
(3) T.J. Davis, D. Gao, T.E. Gurejev, A.W. Stevenson, S.W. Wilkins
   *Phase-contrast imaging of weakly absorbing materials using hard x-rays*
   Nature **373** (1995) p. 595-598

## Claims

1. A method for generating a phase contrast x-ray image (4) of a sample (24), comprising:
a) applying beams (14) of a x-ray source (8) having low coherence to a first beam splitter grating (12) in order to split the beams;
b) applying the splitted beams (16, 18) to a second beam recombiner grating (20) in order to recombine the splitted beams (16, 18) in an analyzer plane (a);
c) introducing the sample (24) either into the splitted beams (16, 18) between the first beam splitter grating (12) and the second beam recombiner grating (20) or into the splitted beams (16, 18) between the second beam recombiner grating (20) and the analyzer plane (a); and
d) measuring the light intensity distribution in or downstream of the analyzer plane (a).

2. The method according to claim 1,
**characterized in that**
the x-ray source (8) comprising an anode material having emissions line in the range of 5 to 50 keV, preferably 15 to 25 keV.

3. The method according to claim 2,
**characterized in that**
the x-ray source (8) comprising a stationary or rotating anode type, selected from a group consisting of Niob, Molybdenum, Rhodium and Palladium.

4. The method according to any one of the preceding claims,
**characterized in that**
the first beam splitter grating (12) is placed at a first distance in the range of 0.1 to 5 m, preferably 0.2 to 2 m, apart from the x-ray source.

5. The method according to claim 4,
**characterized in that**
the first beam splitter grating (12) is of phase shifting type, having a first grating periodicity in the range of 0.1 to 100 micron, preferably 1 to 10 micron.

6. The method according to any one of the preceding claims,
**characterized in that**
the second beam recombiner grating (20) is disposed in a distance in the range of 0.01 to 1 m, preferably 0.05 to 0.5 m, from the first beam splitter grating (12).

7. The method according to claim 6,
**characterized in that**
the second beam recombiner grating (20) is of phase shifting type, having a second grating periodicity which is approximately half of the first grating periodicity.

8. The method according to any one of the preceding claims,
**characterized in that**
a third analyzer grating is disposed in the analyzer plane (a) and is of absorption type, having a third grating periodicity which is substantially equal to the second grating periodicity.

9. The method according to any one of the preceding claims,
**characterized in that**
the first beam splitter grating (12) and the second beam recombiner grating (20) are chosen with respect to its feature height in order to shift the phase in the range of n or an odd multiple of π, with respect to the height preferably in the range of 6 to 45 micron.

10. An apparatus (2) for generating a phase contrast x-ray image (4) comprising in an optical path (6) as seen in the direction of the light flow:
a) an incoherent x-ray source (8);
b) a first beam splitter grating (12) for splitting the light beams (14) of said x-ray source (8);
c) a second beam recombiner grating (20) for recombining the splitted beams (16, 18) in a recombination distance from the second beam recombiner grating (20) in an analyzer plane (a);
d) a third analyzer grating (22) is disposed in an analyzer plane (a) in order to offer an adsorption lines grating matching the interference lines downstream of said second beam recombiner grating (20);
e) an image detector (22) placed downstream of the analyzer plane (a); and
f) a means for introducing a sample (24) into said optical path (6) upstream or downstream of the second beam recombiner grating (20).

11. The apparatus (2) according to claim 10,
**characterized in that**
the x-ray source (8) comprising an anode material having emissions line in the range of 5 to 50 keV, preferably 15 to 25 keV.

12. The apparatus (2) according to claim 11,
**characterized in that**
the x-ray source (8) comprising a stationary or rotating anode type, selected from a group consisting of Niob, Molybdenum, Rhodium and Palladium.

13. The apparatus (2) according to any one of the claims 10 to 12, **characterized in that**
the first beam splitter grating (12) is disposed at a first distance in the range of 0.1 to 5 m, preferably 0.2 to 2 m, apart from the x-ray source (8).

14. The apparatus (2) according to claim 13,
**characterized in that**
the first beam splitter grating (12) is of phase shifting type, having a first grating periodicity in the range of 0.1 to 100 micron, preferably 1 to 10 micron.

15. The apparatus (2) according to any one of the preceding claims, **characterized in that**
the second beam recombiner grating (20) is disposed in a distance in the range of 0.01 to 1 m, preferably 0.05 to 0.5 m, from the first beam splitter grating (12).

16. The apparatus (2) according to claim 15,
**characterized in that**
the second beam recombiner grating (20) is of phase shifting type, having a second grating periodicity which is approximately half of the first grating periodicity.

17. The apparatus (2) according to any one of the claims 10 to 16, **characterized in that**
the third analyzer grating (22) is of absorption type, having a third grating periodicity which is substantially equal to the second grating periodicity.

18. The apparatus (2) according to any one of the claims 10 to 17, **characterized in that**
the first beam splitter grating (12) and the second beam recombiner grating (20) are chosen with respect to its feature height in order to shift the phase in the range of n or an odd multiple of π.

19. The apparatus (2) according to claim 18,
**characterized in that**
the feature height is in the range of 6 to 45 micron.
